# EUROPEAN PATENT APPLICATION

(11) **EP 3 904 520 A1**
(43) Date of publication of application: **03.11.2021**
(21) Application number: 19905665.6
(22) Date of filing: 26.12.2019
(51) Int. Cl.: C12N 15/67, C12N 9/64, C07K 14/55, C07K 14/565, C07K 14/61, C07K 14/805

(54) **GENE EXPRESSION CASSETTE FOR EXPRESSING N-TERMINAL METHIONINE-TRUNCATED PROTEIN OF INTEREST AND METHOD FOR PRODUCING N-TERMINAL METHIONINE-TRUNCATED PROTEIN OF INTEREST BY USING SAME**

(30) Priority: 27.12.2018 KR 20180170531
(71) Applicant: Polus Inc., Incheon 21984 (KR)
(72) Inventor: KIM, Jinhwan, Incheon 21988 (KR); LIM, Hyung Kwon, Gyeonggi-do 13489 (KR); CHOI, Hyoung An, Incheon 21510 (KR); LEE, Eun Bin, Incheon 21997 (KR); LEE, Sang Yeon, Incheon 21568 (KR)
(74) Representative: Budde Schou A/S
(86) International application number: PCT/KR2019/018464
(87) International publication number: WO 2020/138951

(57) **Abstract**

Disclosed are a gene expression cassette for expressing an N-terminal-methionine-truncated target protein comprising a nucleic acid encoding a target protein and a nucleic acid encoding a cysteine protease, in which a cysteine protease recognition sequence is inserted between methionine (Met), which is the first amino acid at the N-terminus of the target protein, and the second amino acid and N-terminal methionine is cleaved with the cysteine protease, and a method of producing an N-terminal-methionine-truncated target protein using the same.

## Description

### [Technical Field]

The present invention relates to a gene expression cassette for expressing an N-terminal-methionine-truncated target protein and a method of producing an N-terminal-methionine-truncated target protein using the same, and more particularly to a gene expression cassette for expressing an N-terminal-methionine-truncated target protein comprising a nucleic acid encoding a target protein and a nucleic acid encoding a cysteine protease, in which a cysteine protease recognition sequence is inserted between methionine (Met), which is the first amino acid at the N-terminus of the target protein, and the second amino acid, so N-terminal methionine is cleaved with the cysteine protease, and a method of producing an N-terminal-methionine-truncated target protein using the same.

### [Background Art]

The production of polypeptides and proteins having biological activity is capable of being utilized for the manufacture of pharmaceutical formulations, enzymes, and other specific chemicals for humans and animals. Recombinant DNA techniques using bacterial cells, fungal cells, and mammalian cells as expression hosts have been employed as particularly useful means for the production of large amounts of polypeptides.

In general, recombinant production of a target protein includes a process of subjecting an expression vector including a signal regulating the expression of a gene to transfection into a host cell when it is operably linked to a gene encoding a protein. The transfected cell grows under conditions suitable for expression of the recombinant protein, and the expressed target protein is recovered, thereby producing the target protein.

The protein thus produced has fewer side effects than typical chemical synthetic drugs and has excellent efficacy against diseases in the human body, resulting in a high clinical trial success rate. However, cutting-edge technologies such as DNA manipulation technology, mass fermentation technology, advanced protein purification technology and the like are required for industrial application thereof. Since the launching of five major protein drugs (insulin, human growth hormone, interferon, EPO, and G-CSF) between 1982 and 1991, thorough research into recombinant protein medicines has been ongoing.

Meanwhile, removal of N-formyl-methionine or methionine (Met), which is a translation initiator, from a recombinant protein such as human hemoglobin, interleukin-2, growth hormone, or the like plays an important role in the function and stability of the recombinant protein. Therefore, many attempts have been made to manufacture a protein in which Met is removed from the N-terminus of a native protein. First, there is a method of using cyanogen bromide to cleave Met under highly acidic conditions, but this may only be applied to proteins having no Met residue therein (Boix et al., 1996). Second, there is a method in which a protease-specific oligopeptide is introduced before a target protein and is then removed by proteases, for example, factor Xa, enterokinase, and cathepsin C (Belagaje et al. 1997). Third, there is a method in which N-terminal Met of the protein may be removed *in vitro* using aminopeptidase of *Aeromonas proteolytica* (Shapiro et al., 1988; Notomista et al., 1999). Fourth, there is a method in which a signal peptide is introduced before a target protein and processed *in vivo* during secretion, but the method has a problem in that the yield is very low (1-5 mg per liter of a culture, Huang et al. 1998).

Against this technical background, the present inventors have made great efforts to develop a novel method for producing an N-terminal-methionine-truncated target protein at high yield, and thus ascertained that, when a cysteine protease recognition sequence is inserted between methionine (Met), which is the first amino acid at the N-terminus of a target protein, and the second amino acid and is expressed along with a cysteine protease, an N-terminal-methionine-truncated target protein may be produced with high efficiency, thereby culminating in the present invention.

### [Disclosure]

It is an object of the present invention to provide a gene expression cassette capable of producing an N-terminal-methionine-truncated target protein and a method of producing a target protein at high yield using the same.

In order to achieve the above and other objects, the present invention provides a gene expression cassette comprising a nucleic acid encoding a target protein and a nucleic acid encoding a cysteine protease, in which a cysteine protease recognition sequence is inserted between methionine (Met), which is the first amino acid at the N-terminus of the target protein, and the second amino acid.

In addition, the present invention provides a recombinant vector into which the gene expression cassette is introduced.

In addition, the present invention provides a recombinant microorganism into which the gene expression cassette is introduced.

In addition, the present invention provides a method of producing a target protein comprising:
(a) producing a target protein from which the first amino acid at the N-terminus, methionine, is cleaved by culturing the recombinant microorganism; and
(b) recovering the produced target protein.

### [Description of Drawings]

FIG. 1 schematically shows the manufacture of a pETDuet-H6-DEVD-hGH-T7-rev-CASP3 vector, which is a gene expression cassette according to an embodiment of the present invention.
FIG. 2 schematically shows the manufacture of a pAPT-Y vector that is used in an embodiment of the present invention.
FIG. 3 schematically shows the manufacture of a pAPT-Y-tag-DEVD-hGH-Lacuv5-rev-CASP3 vector, which is a gene expression cassette according to an embodiment of the present invention.
FIG. 4 shows the configurations of gene expression cassettes according to various embodiments of the present invention.
FIG. 5 shows the cleavage maps of expression vectors according to various embodiments of the present invention.
FIG. 6 shows results of qualitative analysis of expression of a mature growth hormone protein by the pETDuet-H6-DEVD-hGH-T7-rev-CASP vector, which is the gene expression cassette according to an embodiment of the present invention, using SDS-PAGE.
FIG. 7 shows results of qualitative analysis of expression of a mature growth hormone protein by the pAPT-Y-tag-DEVD-hGH-Lacuv5-rev-CASP3 vector, which is the gene expression cassette according to an embodiment of the present invention, using SDS-PAGE.
FIG. 8 shows results of qualitative analysis of expression of a mature growth hormone protein by the pETDuet-H6-DEVD-hGH-T7-rev-CASP3 vector, which is the gene expression cassette according to an embodiment of the present invention, using Western blot.
FIG. 9 shows results of qualitative analysis of expression of a mature growth hormone protein by the pAPT-Y-tag-DEVD-hGH-Lacuv5-rev-CASP3 vector, which is the gene expression cassette according to an embodiment of the present invention, using Western blot.
FIG. 10 shows results of peptide analysis using N-terminal sequencing of the mature growth hormone protein that is expressed by the pETDuet-H6-DEVD-hGH-T7-rev-CASP3 vector, which is the gene expression cassette according to an embodiment of the present invention.
FIG. 11 shows results confirming whether the DNA sequence and the protein sequence match using sequencing for DNA that clones H6-DEVD-hGH, which is a gene expression cassette according to an embodiment of the present invention.
FIG. 12 shows results confirming whether the DNA sequence and the protein sequence match using sequencing for DNA that clones reverse caspase-3, which is a gene expression cassette according to an embodiment of the present invention.
FIG. 13 shows results confirming whether the DNA sequence and the protein sequence match using sequencing for DNA that clones H6-DEVD-hGH, which is a gene expression cassette according to an embodiment of the present invention.
FIG. 14 shows results confirming whether the DNA sequence and the protein sequence match using sequencing for DNA that clones DH6-DEVD-hGH, which is one of gene expression cassettes according to an embodiment of the present invention.
FIG. 15 shows results confirming whether the DNA sequence and the protein sequence match using sequencing for DNA that clones AH6-DEVD-hGH, which is one of gene expression cassettes according to an embodiment of the present invention.
FIG. 16 shows results confirming whether the DNA sequence and the protein sequence match using sequencing for DNA that clones NH6-DEVD-hGH, which is one of gene expression cassettes according to an embodiment of the present invention.
FIG. 17 shows results confirming whether the DNA sequence and the protein sequence match using sequencing for DNA that clones KH6-DEVD-hGH, which is one of gene expression cassettes according to an embodiment of the present invention.
FIG. 18 shows results confirming whether the DNA sequence and the protein sequence match using sequencing for DNA that clones HAK-DEVD-hGH, which is one of gene expression cassettes according to an embodiment of the present invention.
FIG. 19 shows results confirming whether the DNA sequence and the protein sequence match using sequencing for DNA that clones reverse caspase-3 downstream of the Lacuv5 promoter, which is one of gene expression cassettes according to an embodiment of the present invention.

### [Detailed description and preferred embodiment of the invention]

Unless otherwise defined, all technical and scientific terms used herein have the same meanings as those typically understood by those skilled in the art to which the present invention belongs. Generally, the nomenclature used herein and the test method described below are well known in the art and are typical.

In the present invention, in order to produce an N-terminal-methionine-truncated target protein in a recombinant microorganism, a gene expression cassette capable of co-expression in a microorganism by introducing a cysteine protease recognition sequence downstream of N-terminal methionine of a target protein and additionally selecting a cysteine protease is developed, and the effect of the gene expression cassette is verified using a human growth hormone as a representative target protein showing protein activity through cleavage of N-terminal methionine, from which it is found that a mature human growth hormone from which N-terminal methionine is effectively cleaved, even without a separate methionine cleavage process, is expressed at high yield.

Accordingly, an aspect of the present invention pertains to a gene expression cassette comprising a nucleic acid encoding a target protein and a nucleic acid encoding a cysteine protease, in which a cysteine protease recognition sequence is inserted between methionine (Met), which is the first amino acid at the N-terminus of the target protein, and the second amino acid.

In the present invention, the expression of the nucleic acid encoding the target protein and the nucleic acid encoding the cysteine protease may be regulated using a separate promoter, and the promoter may be selected from the group consisting of a T3 promoter, a T5 promoter, a T7 promoter, a tac promoter, a trc promoter, a trp promoter, an arabinose promoter, a Lacuv5 promoter, and a LacI promoter, but is not limited thereto.

In the present invention, the cysteine protease may be reverse caspase-3, reverse caspase-6 (NCBI Accession No. P55212.2), or reverse caspase-9 (NCBI Accession No. BAA82697.1), and is preferably reverse caspase-3, but is not limited thereto.

In the present invention, the cysteine protease recognition sequence may be DEVD, VEID, VEHD or LEHD, but is not limited thereto.

Specifically, in the present invention, when the cysteine protease is reverse caspase-3, the cysteine protease recognition sequence may be DEVD, when the cysteine protease is reverse caspase-6, the cysteine protease recognition sequence may be VEID or VEHD, or when the cysteine protease is reverse caspase-9, the cysteine protease recognition sequence may be LEHD, but the present invention is not limited thereto.

In order to improve the efficiency of expression of the target protein in the present invention, an enhancer may be inserted upstream of a translation initiation codon of the nucleic acid encoding the target protein, and the enhancer may be an A/U-rich sequence, but is not limited thereto.

In the present invention, the target protein may be human hemoglobin, interleukin-2, interferon-beta, or human growth hormone protein, but is not limited thereto, and may be applied to all recombinant proteins that are utilized by cleaving N-terminal methionine or cleaving a specific N-terminal sequence.

In the present invention, a tag may be additionally attached to the N-terminus of the cysteine protease recognition sequence, and the tag may be selected from the group consisting of a His tag, DH6 tag, AH6 tag, NH6 tag, KH6 tag, HAK tag, T7 tag, S-tag, Flag-tag, HA-tag, V5 epitope, PelB, and Xpress epitope, but is not limited thereto. In the present invention, the tag may affect the expression level of the target protein or the cleavage efficiency using cysteine protease depending on the type thereof, and when a human growth hormone is expressed as the target protein, it is confirmed that an N-methionine-truncated human growth hormone is produced at the highest yield when using DH6 or KH6. This is deemed to be due to the different contribution to protein translation stability depending on the type of tag, and the different contribution to the caspase accessibility and the structural stability of the target protein-caspase. Based on the results of analysis *in silico*, it is expected that there will be a difference in the lifespan of mRNA due to a difference in the 5'-site hairpin structure of mRNA, leading to a difference in expression level.

Nucleic acid sequences comprising the tag and the cysteine protease recognition sequence that may be used in the present invention are shown in Table 1 below, but only some examples according to the present invention are listed, and the scope of the present invention is not limited only to these nucleic acid sequences. Meanwhile, in the following sequences, the three nucleic acids (A, T, and G) at the 5' end are the nucleic acid sequence encoding methionine, which is the first amino acid of the target protein.

**[Table 1]**

| | Sequence (5'->3') |
|---|---|
| MRGS-H6-DEVD | ATGAGAGGATCGCATCACCATCACCATCACGACGAGGTGGAT |
| M-H6-DEVD | ATGCATCACCATCACCATCACGACGAGGTGGAT |
| M-DH6-DEVD | ATGGACCATCACCATCACCATCACGACGAGGTGGAT |
| M-AH6-DEVD | ATGGCCCATCACCATCACCATCACGACGAGGTGGAT |
| M-NH6-DEVD | ATGAACCATCACCATCACCATCACGACGAGGTGGAT |
| M-KH6-DEVD | ATGAAACATCACCATCACCATCACGACGAGGTGGAT |
| M-HAK-DEVD | ATGCACGCAAAAGCGCACGCCCACGCACACGCGCATGGCCACGCTCA TGACGAGGTGGAT |

In an embodiment of the present invention, the gene expression cassette may be manufactured in a form comprising a first gene structure in which the first N-terminal amino acid sequence methionine of a human growth hormone, the tag sequence H6, the cysteine protease recognition sequence DEVD, and the second amino acid phenylalanine sequence of the human growth hormone to the last amino acid sequence are operably linked downstream of the T7 promoter, which is the first promoter, and a second gene structure in which reverse caspase-3, which is the cysteine protease, is operably linked downstream of the T7 promoter, which is the second promoter.

In another embodiment of the present invention, the gene expression cassette may be manufactured in a form comprising a first gene structure in which the first N-terminal amino acid sequence methionine of a human growth hormone, the tag H6, the cysteine protease recognition sequence DEVD, and the second amino acid sequence of the human growth hormone to the last amino acid sequence are operably linked downstream of the tac promoter, which is the first promoter, and a second gene structure in which reverse caspase-3, which is the cysteine protease, is operably linked downstream of the Lacuv5 promoter, which is the second promoter.

In still another embodiment of the present invention, the gene expression cassette may be manufactured in a form comprising a first gene structure in which the first N-terminal amino acid sequence methionine of a human growth hormone, the tag DH6, the cysteine protease recognition sequence DEVD, and the second amino acid sequence of the human growth hormone to the last amino acid sequence are operably linked downstream of the tac promoter, which is the first promoter, and a second gene structure in which reverse caspase-3, which is the cysteine protease, is operably linked downstream of the Lacuv5 promoter, which is the second promoter.

In yet another embodiment of the present invention, the gene expression cassette may be manufactured in a form comprising a first gene structure in which the first N-terminal amino acid sequence methionine of a human growth hormone, the tag AH6, the cysteine protease recognition sequence DEVD, and the second amino acid sequence of the human growth hormone to the last amino acid sequence are operably linked downstream of the tac promoter, which is the first promoter, and a second gene structure in which reverse caspase-3, which is the cysteine protease, is operably linked downstream of the Lacuv5 promoter, which is the second promoter.

In a further embodiment of the present invention, the gene expression cassette may be manufactured in a form comprising a first gene structure in which the first N-terminal amino acid sequence methionine of a human growth hormone, the tag NH6, the cysteine protease recognition sequence DEVD, and the second amino acid sequence of the human growth hormone to the last amino acid sequence are operably linked downstream of the tac promoter, which is the first promoter, and a second gene structure in which reverse caspase-3, which is the cysteine protease, is operably linked downstream of the Lacuv5 promoter, which is the second promoter.

In still a further embodiment of the present invention, the gene expression cassette may be manufactured in a form comprising a first gene structure in which the first N-terminal amino acid sequence methionine of a human growth hormone, the tag KH6, the cysteine protease recognition sequence DEVD, and the second amino acid sequence of the human growth hormone to the last amino acid sequence are operably linked downstream of the tac promoter, which is the first promoter, and a second gene structure in which reverse caspase-3, which is the cysteine protease, is operably linked downstream of the Lacuv5 promoter, which is the second promoter.

In yet a further embodiment of the present invention, the gene expression cassette may be manufactured in a form comprising a first gene structure in which the first N-terminal amino acid sequence methionine of a human growth hormone, the tag HAK, the cysteine protease recognition sequence DEVD, and the second amino acid sequence of the human growth hormone to the last amino acid sequence are operably linked downstream of the tac promoter, which is the first promoter, and a second gene structure in which reverse caspase-3, which the cysteine protease, is operably linked downstream of the Lacuv5 promoter, which is the second promoter.

Another aspect of the present invention pertains to a recombinant vector into which the gene expression cassette is introduced.

In the present invention, the gene expression cassette is introduced into a vector that is selected from the group consisting of a pAPT vector, a pETDuet (pETDuet-1) vector, a pET24a(+) vector, a pQE30 vector, a pMAL-c2x vector, a pTrc99a vector, a pBAD vector, a pUC18 vector, and a pUC1 vector, but the present invention is not limited thereto.

Still another aspect of the present invention pertains to a recombinant microorganism into which the gene expression cassette is introduced.

In the present invention, the recombinant vector may be introduced into *E. coli, Corynebacterium glutamicum, Bacillus subtilis,* or yeast, but the present invention is not limited thereto. The *E. coli* that may be used in the present invention may be selected from among all types of *E. coli* such as a K-12 series and a B series capable of inducing overexpression of target proteins, comprising K-12 W3110, B strain C41 (DE3), and B strain Rosetta2 (DE3), and the yeast may be *Pichia pastoris* or *Saccharomyces cerevisiae,* but the range of the recombinant microorganism into which the gene expression cassette according to the present invention may be introduced comprises the entire range of microorganisms available in the art for the production of recombinant proteins, and is not limited to the above exemplary description.

Yet another aspect of the present invention pertains to a method of producing a target protein comprising:
(a) producing a target protein from which the first amino acid at the N-terminus, methionine, is cleaved by culturing the recombinant microorganism; and
(b) recovering the produced target protein.

In the present invention, the produced target protein is preferably a mature human growth hormone. In addition thereto, the target protein to be produced may be N-terminal-methionine-truncated human hemoglobin or interleukin-2. Also, any protein requiring cleavage of N-terminal methionine or N-terminal polypeptide may be utilized as the target protein.

As used herein, the term "gene expression cassette" or simply "expression cassette" refers to a recombinantly or synthetically made nucleic acid construct having a nucleic acid element capable of influencing the expression of a structural gene in a host compatible with the sequence. The expression cassette comprises at least a promoter and optionally a transcription termination signal. Typically, a gene expression cassette comprises a nucleic acid to be transcribed (e.g. a heterologous nucleic acid encoding a target protein (polypeptide)) and a promoter. Additional factors required for or assisting in realizing expression may also be used therewith.

As used herein, the term "promoter (or promoter region)" refers to a portion of DNA of a gene that controls the transcription of DNA to which it is operably linked. The promoter region comprises a specific sequence of DNA sufficient for RNA polymerase to initiate recognition, binding and transcription. Also, such a promoter region comprises a sequence that regulates the recognition, binding and transcription initiation activity of RNA polymerase. The promoter may be constitutive or regulated by a cis-acting or trans-acting factor, depending on the nature of the regulation.

As used herein, the term "operably linked" refers to a functional linkage between a nucleic acid expression control sequence (e.g. a promoter, a signal sequence, or a series of transcription-factor-binding sites) and a heterologous nucleic acid sequence. Here, the expression control sequence affects the transcription and/or translation of a nucleic acid corresponding to the heterologous nucleic acid sequence.

As used herein, the term "recombinant" refers to any material (e.g. a vector or microorganism) that may be formed as a result of genetic manipulation.

As used herein, the term "vector" refers to a DNA product comprising a DNA sequence that is operably linked to a suitable control sequence capable of expressing DNA in a suitable host. The vector may be a plasmid, a phage particle, or a simple potential genomic insert. When transformed into an appropriate host, the vector may replicate and function independently of the host genome, or in some cases may be integrated into the genome itself. Since a plasmid is currently the most commonly used form of the vector, the terms "plasmid" and "vector" are sometimes used interchangeably in the specification of the present invention. For the purposes of the present invention, it is preferred to use a plasmid vector. A typical plasmid vector that may be used for this purpose is configured to comprise (a) a replication origin that allows efficient replication to comprise ones to hundreds of plasmid vectors in each host cell, (b) an antibiotic resistance gene that enables selection of a host cell transformed with a plasmid vector, and (c) a restriction enzyme cleavage site into which a foreign DNA fragment may be inserted. Even when an appropriate restriction enzyme cleavage site does not exist, the vector and foreign DNA may be easily ligated using a synthetic oligonucleotide adapter or linker according to a typical method. After ligation, the vector has to be transformed into an appropriate host cell. Transformation may be easily accomplished using a calcium chloride (CaCl₂) process or an electroporation process (Neumann, et al., EMBO J., 1:841, 1982).

As the vector that is used for overexpression of the gene according to the present invention, an expression vector known in the art may be used. A base sequence is said to be "operably linked" when placed in a functional relationship with another nucleic acid sequence. This may be a gene and control sequence (s) linked in such a way as to enable gene expression when an appropriate molecule (e.g. a transcriptional activation protein) is bound to the control sequence(s). For example, DNA for a pre-sequence or secretory leader is operably linked to DNA for a polypeptide when expressed as a preprotein that participates in the secretion of the polypeptide, a promoter or enhancer is operably linked to a coding sequence when it affects the transcription of the sequence, a ribosome-binding site is operably linked to a coding sequence when it affects transcription of the sequence, or a ribosome-binding site is operably linked to a coding sequence when placed to facilitate translation. In general, "operably linked" means that the linked DNA sequence is in contact therewith, or that a secretory leader is in contact therewith and is present in the reading frame. However, the enhancer need not be in contact therewith. The linking of these sequences is accomplished by ligation (linkage) at a convenient restriction enzyme site. When no such site exists, a synthetic oligonucleotide adapter or linker according to a typical method is used.

As is well known in the art, in order to increase the expression level of a transformed gene in a host cell, the gene has to be operably linked to a transcriptional and translational expression control sequence that functions in the selected expression host. Preferably, the expression control sequence and the corresponding gene are comprised in a single recombinant vector that comprises both a bacterial selection marker and a replication origin.

The recombinant microorganism that is transformed by the recombinant vector described above constitutes another aspect of the present invention. As used herein, the term "transformation" refers to introduction of DNA into a host such that DNA becomes replicable as an extrachromosomal factor or through chromosomal integration.

Here, it is to be understood that not all vectors function equally in expressing the DNA sequence of the present invention. Likewise, not all hosts function equally for the same expression system. However, those skilled in the art will be able to make an appropriate selection from among various vectors, expression control sequences and hosts without undue experimentation and without departing from the scope of the present invention. For example, a vector may be selected in consideration of the host. This is because the vector has to be replicated in the host. The number of copies of a vector, ability to control the number of copies, and expression of another protein encoded by the vector, for example, an antibiotic marker, also have to be taken into consideration.

Moreover, the gene expression cassette according to the present invention may be introduced into the genome of a host cell and may be present as a chromosomal factor. It will be apparent to those skilled in the art to which the present invention belongs that, even when the gene expression cassette is inserted into the genome chromosome of a host cell, it has the same effect as when the recombinant vector is introduced into the host cell as described above.

A better understanding of the present invention may be obtained through the following examples. These examples are merely set forth to illustrate the present invention, and are not to be construed as limiting the scope of the present invention.

### Example 1. Construction of system for overexpression of N-terminal-methionine-truncated target protein

### 1-1. Selection of gene promoter for inducing overexpression of target protein

For system construction, an isopropyl beta-D-1-thiogalactopyranoside (Isopropyl-βIPTG) responsive gene promoter, commonly used in molecular biology research, was selected. Three types of promoters (*tac, T7, Lacuv5)* known to be capable of strongly inducing and regulating gene expression were selected.

### 1-2. Selection of synthetic tag for induction of overexpression of target protein and cleavage of N-terminal methionine

A tag in which five histidines (5x-histidine, H5), a specific amino acid, and a cysteine protease recognition sequence (DEVD) were attached to downstream of N-terminal methionine of a target protein was selected as a basic synthetic tag structure (MHHHHHXaa). Xaa may include lysine, arginine, or histidine, and may also be deleted. In addition, a tag in the form of MXₐₐXₐₐXₐₐXₐₐHHHHHXₐₐ was used by adding a specific amino acid to the N-terminus of the basic synthetic tag. Specifically, 4 types of tags (aspartate-6x-histidine, DH6; alanine-6x-histidine, AH6; asparagine-6x-histidine, NH5; and lysine-6x-histidine, KH6) were manufactured, and a complex histidine synthetic tag (histidine-alanine-lysine-(4x-alanine-histidine)-glycine-histidine-alanine-histidine (HAK)) in which histidine, alanine, and lysine were used in combination was also manufactured.

### 1-3. Selection of cysteine protease for cleavage of N-terminal methionine

Referring to a paper (Srinivasa M. Srinivasula et al., J Biol Chem., 1998 Apr 24;273(17):10107-11) in which subunits of cysteine protease were rebuilt so as to exhibit protein activity even in *E. coli,* a reverse caspase-3 (rev-CASP3) gene was selected. The amino acid sequence and the nucleic acid sequence of reverse caspase-3 that was used in the present invention were as follows.
SEQ ID NO: 1: Amino acid sequence of reverse caspase-3 
MIETDSGVDD DMACHKIPVE ADFLYAYSTA PGYYSWRNSK DGSWFIQSLC AMLKQYADKL 60

EFMHILTRVN RKVATEFESF SFDATFHAKK QIPCIVSMLT KELYFYHDEV DGENTENSVD 120

SKSIKNLEPK IIHGSESMDS GISLDNSYKM DYPEMGLCII INNKNFHKST GMTSRSGTDV 180

DAANLRETFR NLKYEVRNKN DLTREEIVEL MRDVSKEDHS KRSSFVCVLL SHGEEGIIFG 240

TNGPVDLKKI TNFFRGDRCR SLTGKPKLFI IQACRGTELD CGIETD
SEQ ID NO: 2: Nucleic acid sequence of reverse caspase-3

atgatcgaaa ccgatagcgg tgttgatgat gatatggcct gtcataaaat tccggttgaa 60

gccgattttc tgtatgcata tagcaccgca ccgggttatt atagctggcg taatagcaaa 120

gatggcagct ggtttattca gagcctgtgt gcaatgctga aacagtatgc agataaactg 180

gaattcatgc acattctgac ccgtgttaat cgtaaagttg caaccgaatt tgaaagcttt 240

agctttgatg caaccttcca tgccaaaaaa caaattccgt gtattgttag catgctgacc 300

aaagaactgt atttctatca cgatgaagtg gatggcgaaa ataccgaaaa tagcgttgat 360

agcaaaagca tcaaaaacct ggaaccgaaa attatccatg gtagcgaaag catggatagc 420

ggtattagcc tggataacag ctataaaatg gattatccgg aaatgggcct gtgcatcatt 480

atcaacaaca aaaactttca caagagcacc ggtatgacca gccgtagcgg caccgatgtt 540

gatgcagcaa atctgcgtga aacctttcgc aatctgaaat atgaagtgcg caataagaat 600

gatctgacgc gtgaagaaat tgtggaactg atgcgtgatg ttagcaaaga agatcatagc 660

aaacgtagca gctttgtttg tgttctgctg agccatggtg aagaaggtat tatctttggc 720

accaatggtc cggttgatct gaaaaaaatc accaactttt ttcgtggtga tcgttgtcgt 780

agcctgaccg gtaaaccgaa actgtttatc attcaggcat gtcgtggcac cgaactggat 840

tgtggtattg aaaccgatta a

### 1-4. Construction of system for overexpression of target protein

A system for overexpression of a target protein was constructed using the factors selected in Examples 1-1, 1-2, and 1-3. In order to test the system, a gene expression cassette was designed and manufactured for a growth hormone protein (human growth hormone (hGH)). The amino acid sequence and the nucleic acid sequence of the human growth hormone that was used in the present invention were as follows.
SEQ ID NO: 3: Amino acid sequence of human growth hormone 
 60
 120
 180

SEQ ID NO: 4: Nucleic acid sequence of human growth hormone

tttccgacca ttccgctgag ccgtctgttt gataatgcaa tgctgcgtgc acaccgtctg 60

caccagctgg catttgatac ctatcaagaa tttgaagaag cgtatatccc gaaagagcag 120

aaatatagct tcctgcagaa tccgcagacc agcctgtgtt ttagcgaaag cattccgaca 180

ccgagcaatc gtgaagaaac ccagcagaaa agcaatctgg aactgctgcg tattagcctg 240

ctgctgattc agagctggct ggaaccggtg cagtttctgc gtagcgtttt tgcaaatagc 300

ctggtttatg gtgcaagcga tagcaatgtt tatgatctgc tgaaagatct ggaagaaggt 360

attcagaccc tgatgggtcg tctggaagat ggttcaccgc gtaccggtca gatctttaaa 420

cagacctata gcaaattcga taccaacagc cataatgatg atgccctgct gaaaaactat 480

ggtctgctgt attgtttccg caaagatatg gataaagtgg aaacctttct gcgcattgtt 540

cagtgtcgta gcgttgaagg tagctgtggt ttttaa

Meanwhile, a pETDuet-1 plasmid (EMD Millipore Corp.) and a pAPT plasmid (AP Tech.) were used as vectors for cloning.

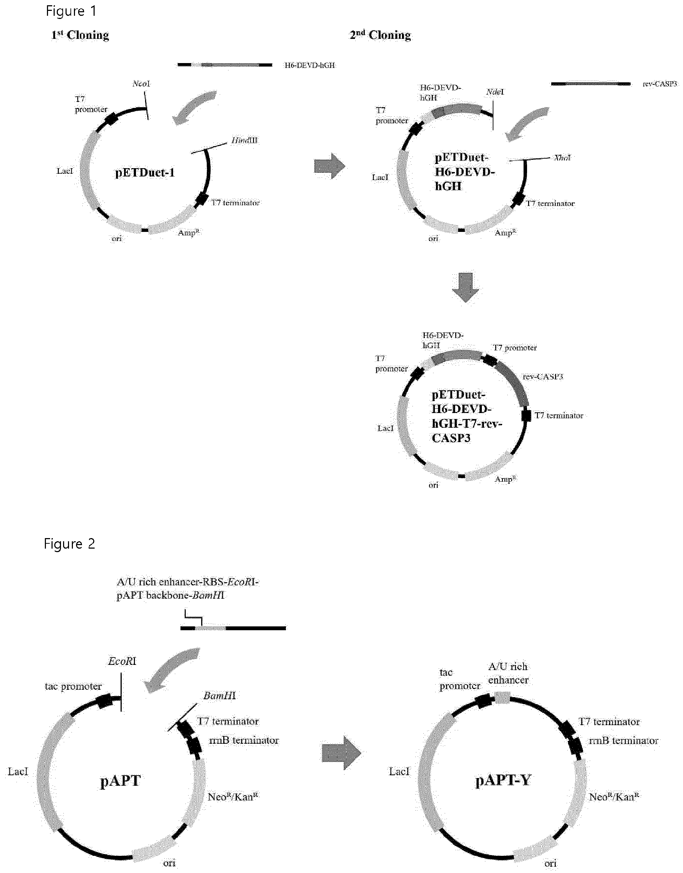

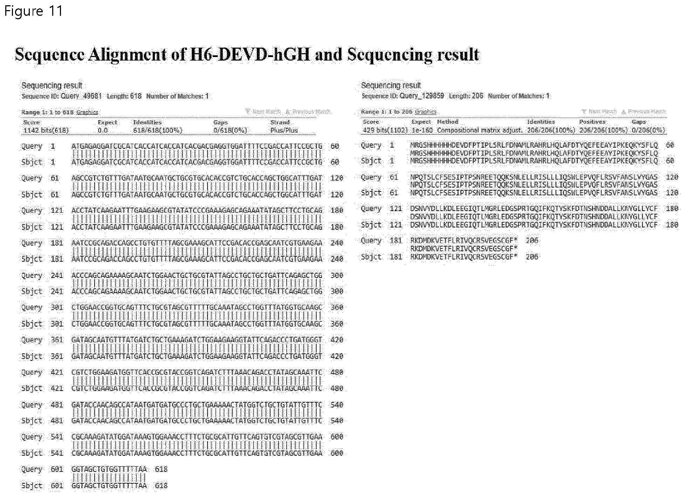

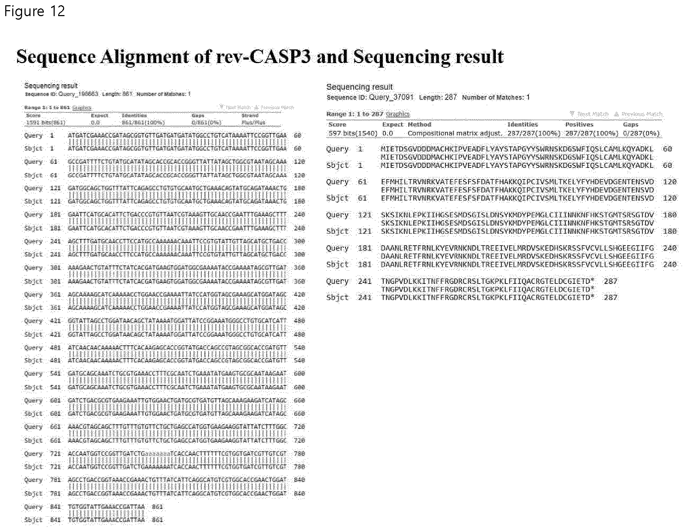

A DNA fragment (H6-DEVD-hGH) was amplified by performing PCR such that the basic synthetic tag (H6-DEVD) to which six histidines and a reverse caspase-3 recognition sequence were linked was attached to DNA encoding the growth hormone protein. The amplified DNA fragment and the pETDuet-1 plasmid cleaved with restriction enzymes *Nco*I/*Hind*III were purified through gel extraction, and were then cloned through homologous recombination. In order to insert the DNA fragment encoding reverse caspase-3, the plasmid subjected to primary cloning was cleaved with restriction enzymes *Nde*I/*Xho*I, and was then secondarily cloned in a homologous recombination manner. The manufacture d plasmid is pETDuet-H6-DEVD-hGH-T7-rev-CASP3, the manufacture thereof is schematically shown in FIG. 1, and the cloning results are shown in FIGS. 11 and 12.

For the pAPT plasmid, an A/U-rich sequence (GCTCTTTAACAATTTATCAGATCCAATAGGAGGAACAAT) as an enhancer for increasing protein translation efficiency was inserted upstream of the translation initiation codon, and the corresponding plasmid was called pAPT-Y. The pAPT-Y cloning is schematically shown in FIG. 2.

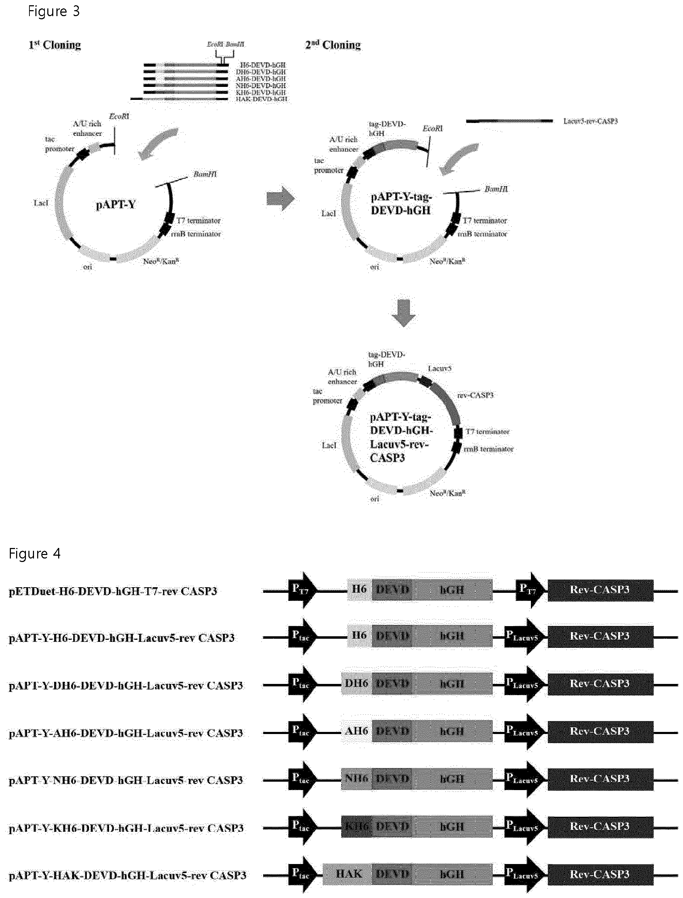

As shown in FIG. 3, DNA encoding the growth hormone protein to which 6 types of tags were attached was amplified through PCR, and was then primarily cloned in a homologous recombination manner to the pAPT-Y plasmid cleaved with restriction enzymes *Eco*RI/*Bam*HI*.* In addition, the Lacuv5 promoter and DNA encoding reverse caspase-3 were secondarily cloned in the above manner to the plasmid subjected to primary cloning so that reverse caspase-3 could be expressed.

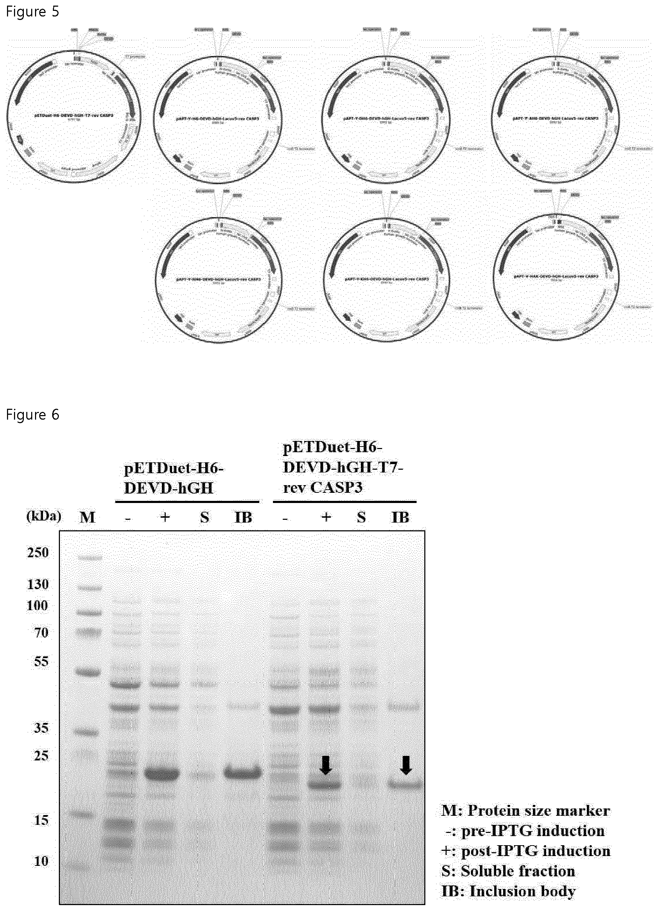

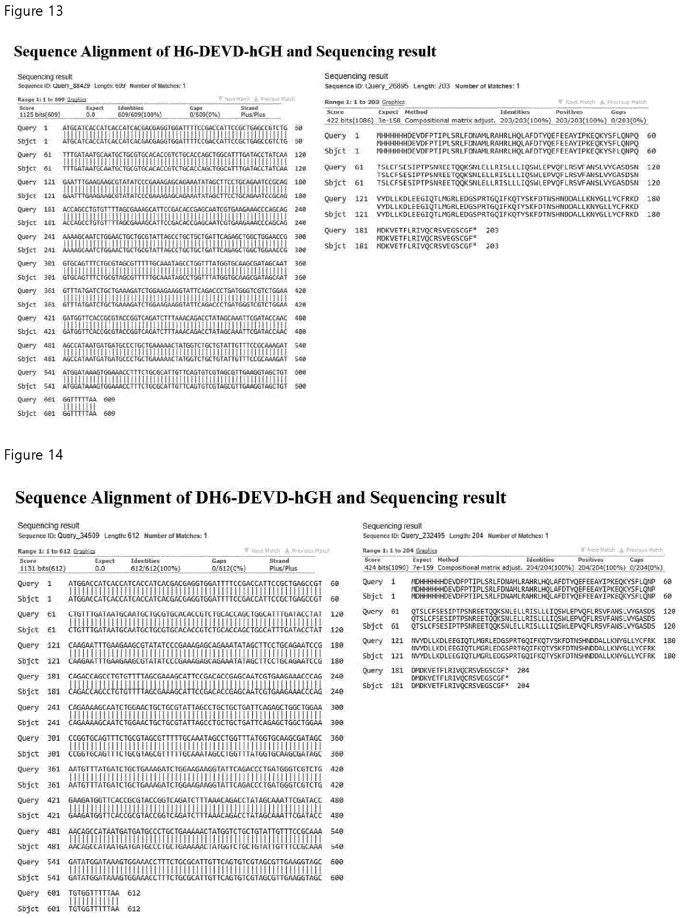

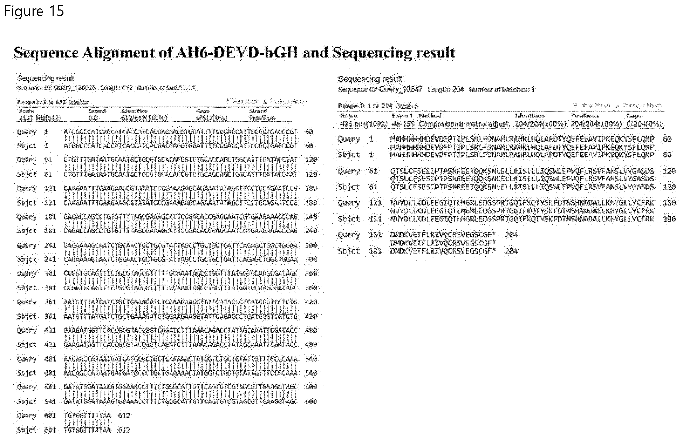

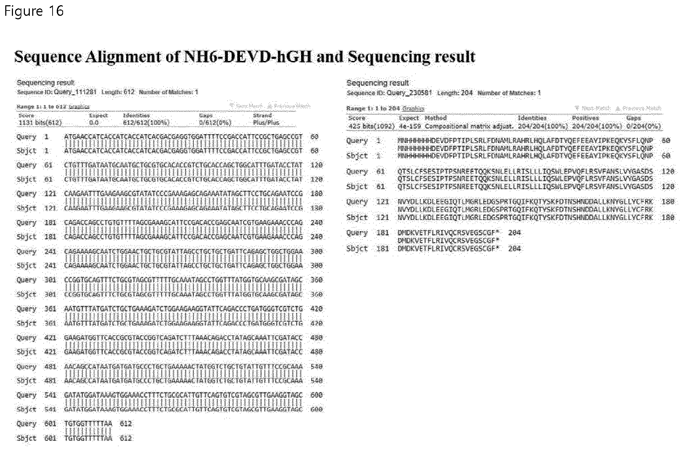

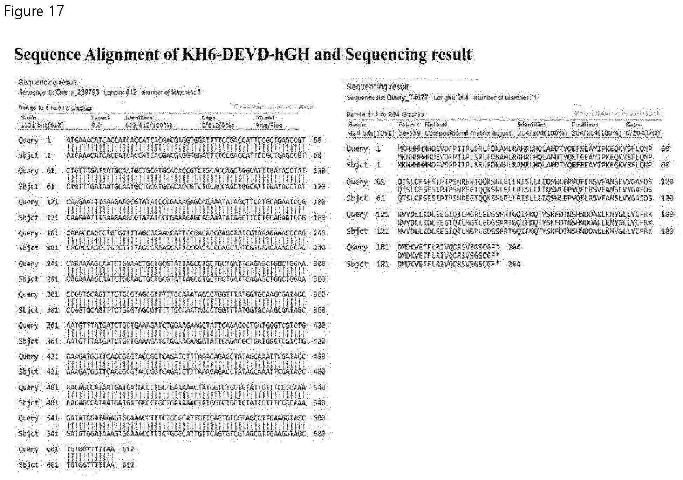

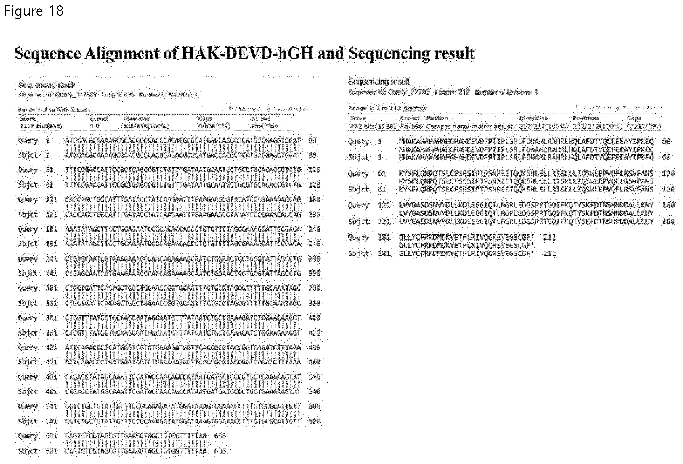

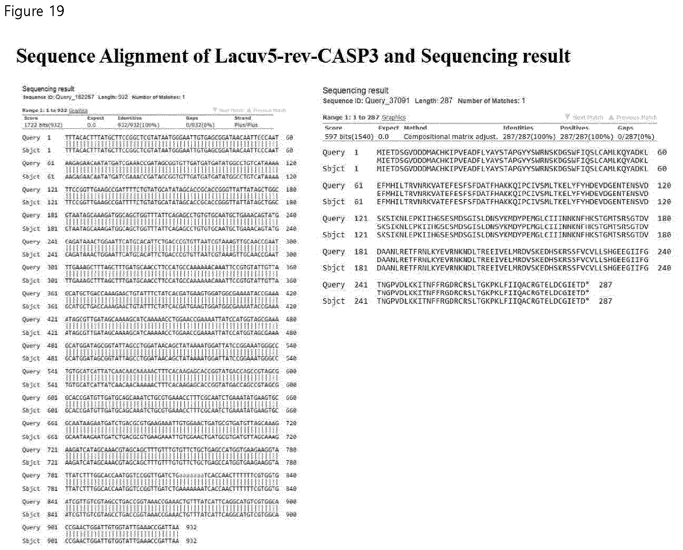

Thereby, pETDuet-H6-DEVD-hGH-T7-rev-CASP3, pAPT-Y-H6-DEVD-hGH-Lacuv5-rev-CASP3, pAPT-Y-DH6-DEVD-hGH-Lacuv5-rev-CASP3, pAPT-Y-AH6-DEVD-hGH-Lacuv5-rev-CASP3, pAPT-Y-NH6-DEVD-hGH-Lacuv5-rev-CASP3, pAPT-Y-KH6-DEVD-hGH-Lacuv5-rev-CASP3, and pAPT-Y-HAK-DEVD-hGH-Lacuv5-rev-CASP3 plasmids were obtained, and are schematically shown in FIG. 4. The results of cloning are shown in FIGS. 13, 14, 15, 16, 17, 18, and 19. The information on the manufactured vectors is shown in FIG. 5.

### Example 2. Evaluation of system for overexpression of N-terminal-methionine-truncated target protein

### 2-1. Evaluation of expression level of N-terminal-methionine-truncated mature growth hormone protein

20 µL of *E. coli (Escherichia coli* K-12 W3110 or *Escherichia coli* C41 (DE3) or *Escherichia coli* Rosetta2 (DE3)) manufactured to enable transformation was dispensed in an amount of 1.0×10⁶ cells or more into each tube, mixed with 10 ng of the plasmid manufactured above, and then allowed to stand on ice for 5 minutes. The E. *coli-DNA* mixture was transferred into a 0.2 cm cuvette for electroporation, current at 2.5 kV was applied thereto, 500 µL of a SOC medium (super optimal broth with catabolite repression) was mixed therewith, and the mixture was cultured at 37°C for 30 minutes. 100 µL of the culture solution was spread on an LB (Luria-Bertani) solid medium (agar plate) containing a 100 µg/mL kanamycin antibiotic, followed by stationary culture at 37°C for 16 hours to thus complete transformation. The strains showing resistance to kanamycin were randomly selected, inoculated in 5 mL of an LB liquid medium (100 µg/mL), and then cultured at 37°C. When the optical density (OD) of the culture solution was measured to be 0.8-1.3 at a wavelength of 600 nm, 1 mM IPTG (Isopropyl β) was added to the culture solution to induce expression of the growth hormone protein and reverse caspase-3 for 3 hours. The expression levels of growth hormone protein and reverse caspase-3 were calculated using SDS-PAGE (sodium dodecyl sulfate-polyacrylamide gel electrophoresis) and Bio-Rad Image Lab software version 5.2.1.

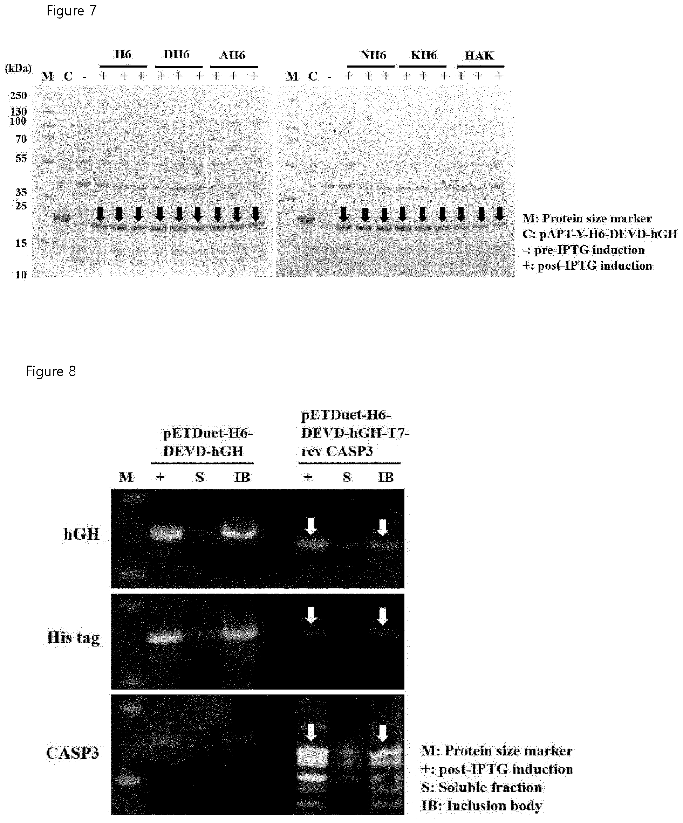

Compared with the strain in which N-terminal methionine was maintained due to the absence of reverse caspase-3, it was confirmed that expression of the growth hormone protein from which N-terminal methionine was removed was induced when expressed along with reverse caspase-3 (FIGS. 6 and 7). The strain having the highest expression rate in the expressed growth hormone protein in FIG. 7 was analyzed for each synthetic tag, and the results thereof are shown in Table 2 below. Reverse caspase-3 exhibited an expression rate of 1.0-1.3%.

**[Table 2]**

| Tag | Expression Rate (%) | |
|---|---|---|
| | M-tag-hGH | Cleaved hGH |
| H6 | 1.0 | 41.4 |
| DH6 | 1.0 | 52.1 |
| AH 6 | 1.1 | 45.9 |
| NH6 | 1.2 | 47.2 |
| KH6 | 1. 6 | 51.1 |
| HAK | 1.2 | 27.1 |

### 2-2. Evaluation of cleavage of N-terminal methionine and synthetic tag 1

Western blot was performed on the growth hormone protein and the synthetic tag in order to confirm whether N-terminal methionine was cleaved.

Cells in which protein expression was induced through IPTG were collected and then lysed ten times each for 10 seconds at 20% intensity (amplitude) using a cell lysis buffer (50 mM Tris-HCl (pH 8.5), 10 mM EDTA) and a sonicator. The lysed cells were centrifuged at 12,000 × g at 4°C for 30 minutes to afford a soluble protein and an insoluble protein (inclusion body). Protein electrophoresis was performed by loading total protein and soluble and insoluble proteins on a 4-12% Bis-Tris gel under the same conditions as in SDS-PAGE, after which protein transfer was carried out using a PVDF membrane (polyvinylidene fluoride membrane). Blocking was performed in order to reduce nonspecific binding to the membrane, and binding of primary antibodies anti-His, anti-hGH, and anti-CASP3 was induced. A secondary antibody to which a fluorescent factor was attached was allowed to react therewith, after which the presence or absence of the growth hormone protein, synthetic tag, and reverse caspase-3 was observed using an Odyssey Infrared Imaging System. The results of Western blot were analyzed using Li-COR Image studio analysis software version 3.1.

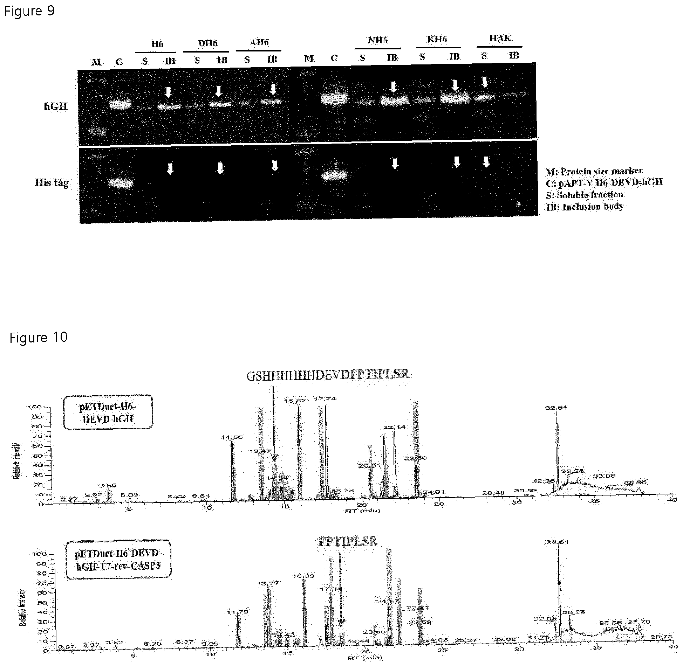

Compared with the strain in which N-terminal methionine was maintained due to the absence of reverse caspase-3, it was confirmed that 96.12% of the N-terminal methionine of the growth hormone protein and the synthetic tag was cleaved when expressed along with reverse caspase-3 (FIG. 8). These results were the same regardless of the type of synthetic tag, and in FIG. 9, it was confirmed that 99.30% or more of the synthetic tag was cleaved.

### 2-3. Evaluation of cleavage of N-terminal methionine and synthetic tag 2

N-terminal sequencing was performed on the growth hormone protein and the synthetic tag in order to confirm whether N-terminal methionine was cleaved. The insoluble protein of Example 2-2 was loaded on a 4-12% Bis-Tris gel and SDS-PAGE was performed, after which the growth hormone protein was identified and acquired through staining with Coomassie blue. The protein thus obtained was subjected to trypsinization (trypsin-based enzymatic digestion), followed by N-terminal sequencing through liquid chromatography-mass spectrometry (LC-MS). Here, the liquid chromatography column that was used was an Acclaim RSLC 120, C18, 2.2 µm Analytical (2.1 mm × 25 cm; 2.2 µm packing C18), the mobile phase A was 0.1% formic acid in water, the mobile phase B was 0.1% formic acid in acetonitrile, and analysis was performed under UV radiation at 214 nm. Mass spectrometry was performed in the range of 300-1,900 m/z using a Thermo Q-Exactive Plus-Quad-Orbitrap MS system.

For the strain in which N-terminal methionine was maintained due to the absence of reverse caspase-3, the 'GSHHHHHHDEVDFPTIPLSR' sequence to which the synthetic tag was attached was identified. For the strain expressed along with reverse caspase-3, the 'FPTIPLSR' sequence, in which the N-terminal methionine of the growth hormone protein and the synthetic tag were cleaved, was identified (FIG. 10).

From the above description, those skilled in the art to which the present invention belongs will appreciate that the present invention may be embodied in other specific forms without changing the technical spirit or essential features thereof. In this regard, the embodiments described above should be understood to be non-limiting and illustrative in every way. The scope of the present invention is defined by the claims below rather than the aforementioned detailed description, and all changes or modified forms that are capable of being derived from the meaning, range, and equivalent concepts of the appended claims should be construed as being comprised in the scope of the present invention.

### [Industrial Applicability]

When a gene expression cassette according to the present invention is used, N-terminal methionine of a target protein is cleaved by a cysteine protease that is introduced along with the target protein, so methionine is removed without a separate methionine removal process, thus making it possible to simplify the process of producing a target protein and to produce a target protein having activity at high yield.

### [Sequence List Free Text]

An electronic file is attached.

## Claims

1. A gene expression cassette comprising a nucleic acid encoding a target protein and a nucleic acid encoding a cysteine protease, wherein a cysteine protease recognition sequence is inserted between methionine (Met) which is a first amino acid at an N-terminus of the target protein and a second amino acid.

2. The gene expression cassette according to claim 1, wherein expression of the nucleic acid encoding the target protein and the nucleic acid encoding the cysteine protease is regulated by a separate promoter.

3. The gene expression cassette according to claim 2, wherein the promoter is selected from the group consisting of a T3 promoter, a T5 promoter, a T7 promoter, a tac promoter, a trc promoter, a trp promoter, an arabinose promoter, a Lacuv5 promoter, and a LacI promoter.

4. The gene expression cassette according to claim 1, wherein the cysteine protease recognition sequence is DEVD, VEID, VEHD, or LEHD.

5. The gene expression cassette according to claim 1, wherein the cysteine protease is reverse caspase-3, reverse caspase-6, or reverse caspase-9.

6. The gene expression cassette according to claim 1, wherein an enhancer is inserted upstream of a translation initiation codon of the nucleic acid encoding the target protein.

7. The gene expression cassette according to claim 6, wherein the enhancer is an A/U-rich sequence.

8. The gene expression cassette according to claim 1, wherein the target protein is human hemoglobin, interleukin-2, interferon-beta, or human growth hormone.

9. The gene expression cassette according to claim 1, wherein a tag is additionally attached to an N-terminus of the cysteine protease recognition sequence.

10. The gene expression cassette according to claim 9, wherein the tag is selected from the group consisting of a His tag, DH6 tag, AH6 tag, NH6 tag, KH6 tag, HAK tag, T7 tag, S-tag, Flag-tag, HA-tag, V5 epitope, PelB, and Xpress epitope.

11. A recombinant vector into which the gene expression cassette according to any one of claims 1 to 10 is introduced.

12. The recombinant vector according to claim 11, wherein the gene expression cassette is introduced into a vector selected from the group consisting of a pAPT vector, a pETDuet(pETDuet-1) vector, a pET24a(+) vector, a pQE30 vector, a pMAL-c2x vector, a pTrc99a vector, a pBAD vector, a pUC18 vector, and a pUC19 vector.

13. A recombinant microorganism into which the gene expression cassette according to any one of claims 1 to 10 is introduced.

14. The recombinant microorganism according to claim 13, wherein the microorganism is *Escherichia coli*, *Corynebacterium glutamicum*, *Bacillus subtilis*, or yeast.

15. A method of producing a target protein comprising:
(a) producing a target protein from which a first amino acid at an N-terminus, methionine, is cleaved by culturing the recombinant microorganism according to claim 14; and
(b) recovering the produced target protein.

16. The method according to claim 15, wherein the produced target protein is a mature human growth hormone.


<110>    POLUS Inc.

<120>    Gene Expression Cassette for Expressing a Target Protein of which
         Methionine at the N-terminus is Cleaved and Method for Producing
         the Target Protein Using the Same

<130>    PP-B2346

<150>    KR 10-2018-0170531
<151>    2018-12-27

<160>    11

<170>    KoPatentIn 3.0

<210>    1
<211>    286
<212>    PRT
<213>    Homo sapiens

<400>    1
Met Ile Glu Thr Asp Ser Gly Val Asp Asp Asp Met Ala Cys His Lys
  1               5                  10                  15

Ile Pro Val Glu Ala Asp Phe Leu Tyr Ala Tyr Ser Thr Ala Pro Gly
             20                  25                  30

Tyr Tyr Ser Trp Arg Asn Ser Lys Asp Gly Ser Trp Phe Ile Gln Ser
         35                  40                  45

Leu Cys Ala Met Leu Lys Gln Tyr Ala Asp Lys Leu Glu Phe Met His
     50                  55                  60

Ile Leu Thr Arg Val Asn Arg Lys Val Ala Thr Glu Phe Glu Ser Phe
 65                  70                  75                  80

Ser Phe Asp Ala Thr Phe His Ala Lys Lys Gln Ile Pro Cys Ile Val
                 85                  90                  95

Ser Met Leu Thr Lys Glu Leu Tyr Phe Tyr His Asp Glu Val Asp Gly
            100                 105                 110

Glu Asn Thr Glu Asn Ser Val Asp Ser Lys Ser Ile Lys Asn Leu Glu
        115                 120                 125

Pro Lys Ile Ile His Gly Ser Glu Ser Met Asp Ser Gly Ile Ser Leu
    130                 135                 140

Asp Asn Ser Tyr Lys Met Asp Tyr Pro Glu Met Gly Leu Cys Ile Ile
145                 150                 155                 160

Ile Asn Asn Lys Asn Phe His Lys Ser Thr Gly Met Thr Ser Arg Ser
                165                 170                 175

Gly Thr Asp Val Asp Ala Ala Asn Leu Arg Glu Thr Phe Arg Asn Leu
            180                 185                 190

Lys Tyr Glu Val Arg Asn Lys Asn Asp Leu Thr Arg Glu Glu Ile Val
        195                 200                 205

Glu Leu Met Arg Asp Val Ser Lys Glu Asp His Ser Lys Arg Ser Ser
    210                 215                 220

Phe Val Cys Val Leu Leu Ser His Gly Glu Glu Gly Ile Ile Phe Gly
225                 230                 235                 240

Thr Asn Gly Pro Val Asp Leu Lys Lys Ile Thr Asn Phe Phe Arg Gly
                245                 250                 255

Asp Arg Cys Arg Ser Leu Thr Gly Lys Pro Lys Leu Phe Ile Ile Gln
            260                 265                 270

Ala Cys Arg Gly Thr Glu Leu Asp Cys Gly Ile Glu Thr Asp
        275                 280                 285

<210>    2
<211>    861
<212>    DNA
<213>    Homo sapiens

<400>    2
atgatcgaaa ccgatagcgg tgttgatgat gatatggcct gtcataaaat tccggttgaa      60

gccgattttc tgtatgcata tagcaccgca ccgggttatt atagctggcg taatagcaaa     120

gatggcagct ggtttattca gagcctgtgt gcaatgctga aacagtatgc agataaactg     180

gaattcatgc acattctgac ccgtgttaat cgtaaagttg caaccgaatt tgaaagcttt     240

agctttgatg caaccttcca tgccaaaaaa caaattccgt gtattgttag catgctgacc     300

aaagaactgt atttctatca cgatgaagtg gatggcgaaa ataccgaaaa tagcgttgat     360

agcaaaagca tcaaaaacct ggaaccgaaa attatccatg gtagcgaaag catggatagc     420

ggtattagcc tggataacag ctataaaatg gattatccgg aaatgggcct gtgcatcatt     480

atcaacaaca aaaactttca caagagcacc ggtatgacca gccgtagcgg caccgatgtt     540

gatgcagcaa atctgcgtga aacctttcgc aatctgaaat atgaagtgcg caataagaat     600

gatctgacgc gtgaagaaat tgtggaactg atgcgtgatg ttagcaaaga agatcatagc     660

aaacgtagca gctttgtttg tgttctgctg agccatggtg aagaaggtat tatctttggc     720

accaatggtc cggttgatct gaaaaaaatc accaactttt ttcgtggtga tcgttgtcgt     780

agcctgaccg gtaaaccgaa actgtttatc attcaggcat gtcgtggcac cgaactggat     840

tgtggtattg aaaccgatta a                                               861

<210>    3
<211>    191
<212>    PRT
<213>    Homo sapiens

<400>    3

<210>    4
<211>    576
<212>    DNA
<213>    Homo sapiens

<400>    4
tttccgacca ttccgctgag ccgtctgttt gataatgcaa tgctgcgtgc acaccgtctg        60

caccagctgg catttgatac ctatcaagaa tttgaagaag cgtatatccc gaaagagcag       120

aaatatagct tcctgcagaa tccgcagacc agcctgtgtt ttagcgaaag cattccgaca       180

ccgagcaatc gtgaagaaac ccagcagaaa agcaatctgg aactgctgcg tattagcctg       240

ctgctgattc agagctggct ggaaccggtg cagtttctgc gtagcgtttt tgcaaatagc       300

ctggtttatg gtgcaagcga tagcaatgtt tatgatctgc tgaaagatct ggaagaaggt       360

attcagaccc tgatgggtcg tctggaagat ggttcaccgc gtaccggtca gatctttaaa       420

cagacctata gcaaattcga taccaacagc cataatgatg atgccctgct gaaaaactat       480

ggtctgctgt attgtttccg caaagatatg gataaagtgg aaacctttct gcgcattgtt       540

cagtgtcgta gcgttgaagg tagctgtggt ttttaa                                 576

<210>    5
<211>    42
<212>    DNA
<213>    Artificial Sequence

<220>
<223>    MRGS-H6-DEVD


<400>    5
atgagaggat cgcatcacca tcaccatcac gacgaggtgg at                          42


<210>    6
<211>    33
<212>    DNA
<213>    Artificial Sequence

<220>
<223>    M-H6-DEVD


<400>    6
atgcatcacc atcaccatca cgacgaggtg gat                                    33


<210>    7
<211>    36
<212>    DNA
<213>    Artificial Sequence

<220>
<223>    M-DH6-DEVD


<400>    7
atggaccatc accatcacca tcacgacgag gtggat                                 36


<210>    8
<211>    36
<212>    DNA
<213>    Artificial Sequence

<220>
<223>    M-AH6-DEVD


<400>    8
atggcccatc accatcacca tcacgacgag gtggat                                 36


<210>    9
<211>    36
<212>    DNA
<213>    Artificial Sequence

<220>
<223>    M-NH6-DEVD


<400>    9
atgaaccatc accatcacca tcacgacgag gtggat                                 36


<210>    10
<211>    36

<212>    DNA
<213>    Artificial Sequence

<220>
<223>    M-KH6-DEVD


<400>    10
atgaaacatc accatcacca tcacgacgag gtggat                              36


<210>    11
<211>    60
<212>    DNA
<213>    Artificial Sequence

<220>
<223>    M-HAK-DEVD


<400>    11
atgcacgcaa aagcgcacgc ccacgcacac gcgcatggcc acgctcatga cgaggtggat        60

                                                                          60